# EUROPEAN PATENT SPECIFICATION

(11) **EP 3 390 363 B1**
(45) Date of publication and mention of the grant of the patent: **18.09.2019**
(21) Application number: 16809679.0
(22) Date of filing: 01.12.2016
(51) Int. Cl.: C07D 213/82, A61Q 19/02, A61K 8/49

(54) **PROCESS FOR DEPIGMENTING KERATIN MATERIALS USING THIOPYRIDINONE COMPOUNDS**
VERFAHREN ZUR DEPIGMENTIERUNG VON KERATINMATERIALIEN MITHILFE VON THIOPYRIDINONVERBINDUNGEN
PROCÉDÉ DE DÉPIGMENTATION DE MATIÈRES KÉRATINIQUES AU MOYEN DE COMPOSÉS DE THIOPYRIDINONE

(30) Priority: 18.12.2015 FR 1562774
(43) Date of publication of application: 24.10.2018
(73) Proprietor: L'OREAL, 75008 Paris (FR)
(72) Inventor: MARAT, Xavier, 93601 Aulnay-sous-Bois (FR); GUEGUINIAT, Amélie, 93601 Aulnay-sous-Bois (FR); GREGOIRE, Sébastien, 93601 Aulnay-sous-Bois (FR); XU, Jinzhu, 93601 Aulnay-sous-Bois (FR); GUERREIRO, Patricio, 93601 Aulnay-sous-Bois (FR)
(74) Representative: L'Oreal
(86) International application number: PCT/EP2016/079394
(87) International publication number: WO 2017/102349

(56) References cited:
- WO-A1-2012/080075

## Description

The present invention relates to a cosmetic treatment process in particular for depigmenting and/or bleaching the skin, using at least one compound of thiopyridinone type.

At various periods of their life, some people see the appearance on their skin, and more in particular on the hands, of darker and/or more coloured spots, which give the skin heterogeneity. These spots are in particular due to a high concentration of melanin in the keratinocytes located at the surface of the skin.

The use of harmless topical depigmenting substances with good efficacy is most particularly desired for the purpose of treating pigmentation spots.

The mechanism of formation of skin pigmentation, i.e. the formation of melanin, is particularly complex and schematically involves the following main steps: Tyrosine ---> Dopa ---> Dopaquinone ---> Dopachrome ---> Melanin Tyrosinase (monophenol dihydroxyl phenylalanine: oxygen oxidoreductase EC 1.14.18.1) is the essential enzyme involved in this sequence of reactions. It in particular catalyzes the reaction for conversion of tyrosine into dopa (dihydroxyphenylalanine) by means of its hydroxylase activity, and the reaction for conversion of dopa into dopaquinone by means of its oxidase activity. This tyrosinase acts only when it is in mature form under the action of certain biological factors.

A substance is acknowledged as being depigmenting if it acts directly on the vitality of the epidermal melanocytes where melanogenesis takes place, and/or if it interferes with one of the steps of melanin biosynthesis, either by inhibiting one of the enzymes involved in melanogenesis, or by inserting itself as a structural analogue of one of the chemical compounds of the melanin synthesis chain, which chain can then be blocked, thus ensuring depigmentation.

Arbutin, niacinamide and kojic acid are known as skin depigmenting agents.

Substances have been sought which have an effective depigmenting action, in particular greater than that of arbutin, niacinamide and kojic acid.

In this regard, the Applicant has discovered, surprisingly and unexpectedly, that certain thiopyridinone compounds have good depigmenting activity, even at low concentration.

A subject of the invention is thus a non-therapeutic cosmetic process for depigmenting, lightening and/or bleaching keratin materials, in particular the skin, comprising the application of a cosmetic composition comprising, in a physiologically acceptable medium, at least one compound of formula (I) as defined below.

The invention also relates to the non-therapeutic cosmetic use of a compound of formula (I) as an agent for bleaching, lightening and/or depigmenting keratin materials, in particular the skin.

The compounds used according to the invention can efficiently depigment and/or lighten, or even bleach, human skin. They are in particular intended to be applied to the skin of individuals bearing brownish pigmentation spots or senescence spots, or to the skin of individuals wishing to combat the appearance of a brownish colour caused by melanogenesis.

They may also make it possible to depigment and/or lighten bodily hairs, the eyelashes, head hair, and also the lips and/or the nails.

A subject of the invention is also the cosmetic use of a compound of formula (I) as described previously, as an agent for bleaching and/or depigmenting the skin, bodily hairs, the eyelashes or head hair, and also the lips and/or the nails, and preferably the skin, in particular for eliminating pigmentation spots or senescence spots, and/or as anti-tanning agents.

The compounds used according to the invention therefore correspond to formula (I) below: 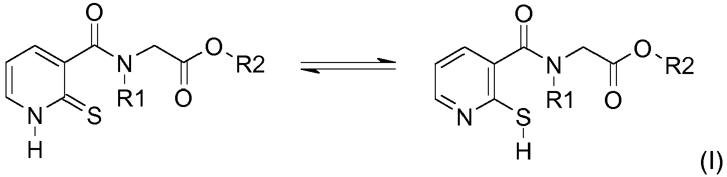 in which:
R1 denotes a radical chosen from:
   a) a hydrogen atom;
   b) a saturated linear C₁-C₆ alkyl group;
R2 denotes a radical chosen from:
   a) a hydrogen atom;
   b) a saturated linear C₁-C₁₀ alkyl group;
   c) a saturated branched C₃-C₁₀ alkyl group;
   d) a C₁-C₆ phenylalkyl group such as benzyl,
and also the tautomers thereof, the salts thereof, the solvates thereof and the optical isomers thereof, and the racemates thereof, alone or as a mixture.

The salts of the compounds of formula (I) comprise the conventional non-toxic salts of said compounds, such as those formed from an acid or base.

As salts of the compounds of formula (I), mention may be made of:
the salts obtained by addition of the compound of formula (I) (when it comprises an acid group) to a mineral base, such as sodium hydroxide, potassium hydroxide, calcium hydroxide, ammonium hydroxide, magnesium hydroxide, lithium hydroxide, and sodium, potassium or calcium carbonate or hydrogen carbonate for example;
or to an organic base such as a primary, secondary or tertiary alkylamine, for example triethylamine or butylamine. This primary, secondary or tertiary alkylamine may comprise one or more nitrogen and/or oxygen atoms and may thus comprise, for example, one or more alcohol functions; mention may be made in particular of 2-amino-2-methylpropanol, ethanolamine, triethanolamine, 2-dimethylaminopropanol, 2-amino-2-(hydroxymethyl)-1,3-propanediol and 3-(dimethylamino)propylamine.

Mention may also be made of the salts of amino acids, for instance lysine, arginine, guanidine, glutamic acid and aspartic acid. Advantageously, the salts of the compounds of formula (I) (when it comprises an acid group) may be chosen from alkali metal or alkaline-earth metal salts such as sodium, potassium, calcium or magnesium salts; ammonium salts.

The acceptable solvates of the compounds described in the present invention comprise conventional solvates such as those formed during the preparation of said compounds owing to the presence of solvents. Mention may be made, by way of example, of the solvates due to the presence of water or of linear or branched alcohols, such as ethanol or isopropanol.

The optical isomers are in particular the enantiomers and the diastereoisomers.

Preferentially, the linear or branched groups may be chosen from methyl, ethyl, propyl, isopropyl, butyl, isobutyl and tert-butyl, pentyl, hexyl, heptyl, octyl, nonyl and decyl.

More preferentially, the saturated linear or branched alkyl groups may be chosen from methyl, ethyl, propyl, isopropyl, butyl, isobutyl and tert-butyl, pentyl, hexyl, heptyl and octyl.
The compound a) is disclosed in the PubCHEM database (No. 47329290) http://pubchem.ncbi.nlm.nih.gov/compound/47329290?from=summary#section= Top entry : 2010-11-26
The compound b) CAS> 1240664-41-8 is described in the publication:
   Synthesis of N-(2-mercaptopyridyl-3-formyl)-N-alkyl glycine and the corresponding disulfides
   Luo, Y. L.; Yang, Z. X.; Peng, S. X.
   Div. Med. Chem., China Pharm. Univ., Nanjing, 210009, Peop. Rep. China Yaoxue Xuebao (1990), 25(5), 374-8.

Preferably, the compounds of formula (I) have the following meanings:
R1 denotes a radical chosen from:
   a) a hydrogen atom;
   b) a saturated linear C₁-C₄ alkyl radical and preferably methyl;
R2 denotes a radical chosen from:
   a) a hydrogen atom;
   b) a saturated linear C₁-C₆ alkyl group;
   c) a saturated branched C₃-C₆ alkyl group,
and also the tautomers thereof, the salts thereof, the solvates thereof and the optical isomers thereof, and the racemates thereof, alone or as a mixture.

More particularly, the compounds of formula (I) have the following meanings:
R1 denotes a radical chosen from:
   a) a hydrogen atom;
   b) a methyl radical;
R2 denotes a radical chosen from:
   a) a hydrogen atom;
   b) a saturated linear C₁-C₄ alkyl group, preferably ethyl;
   c) a saturated branched C₃-C₄ alkyl group, preferably isopropyl and isobutyl,
and also the tautomers thereof, the salts thereof, the solvates thereof and the optical isomers thereof, and the racemates thereof, alone or as a mixture.

### Novel compounds

Another subject of the invention is the novel compounds of formula (II): 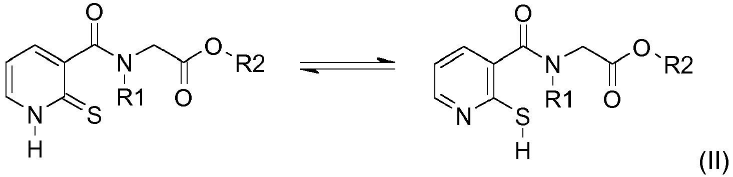 in which:
R1 denotes a radical chosen from:
   a) a hydrogen atom;
   b) a saturated linear C₁-C₆ alkyl group;
R2 denotes a radical chosen from:
   a) a hydrogen atom;
   b) a saturated linear C₁-C₁₀ alkyl group;
   c) a saturated branched C₃-C₁₀ alkyl group;
   d) a C₁-C₆ phenylalkyl group such as benzyl,
and also the tautomers thereof, the salts thereof, the solvates thereof and the optical isomers thereof, and the racemates thereof, alone or as a mixture,
with the exception of the following two compounds (a) and (b) and of the tautomers thereof:

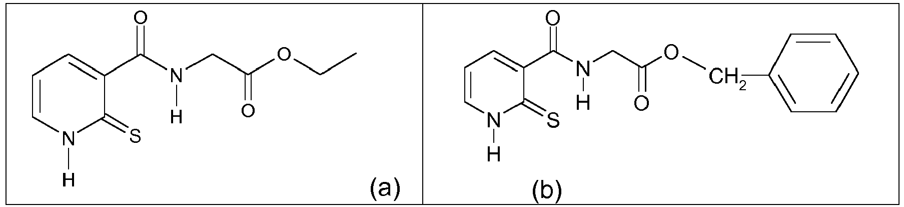

Preferably, the compounds of formula (II) have the following meanings:
R1 denotes a radical chosen from:
   a) a hydrogen atom;
   b) a saturated linear C₁-C₄ alkyl radical and preferably methyl;
R2 denotes a radical chosen from:
   a) a hydrogen atom;
   b) a saturated linear C₁-C₆ alkyl group;
   c) a saturated branched C₃-C₆ alkyl group,
and also the tautomers thereof, the salts thereof, the solvates thereof and the optical isomers thereof, and the racemates thereof, alone or as a mixture,
with the exception of the following compound (a) and of the tautomer thereof:

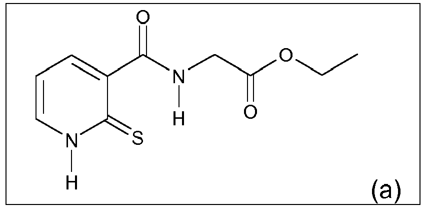

More particularly, the compounds of formula (II) have the following meanings:
R1 denotes a radical chosen from:
   a) a hydrogen atom;
   b) a methyl radical;
R2 denotes a radical chosen from:
   a) a hydrogen atom;
   b) a saturated linear C₁-C₄ alkyl group, preferably ethyl;
   C) a saturated branched C₃-C₄ alkyl group, preferably isopropyl and isobutyl,
and also the tautomers thereof, the salts thereof, the solvates thereof and the optical isomers thereof, and the racemates thereof, alone or as a mixture,
with the exception of the following compound (a) and of the tautomer thereof:

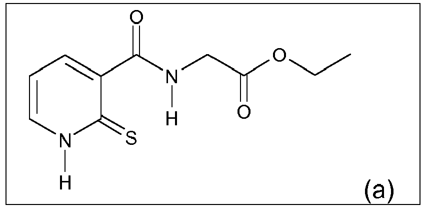

Among the compounds of formula (I) or (II), the following compounds are preferably used:

| Structure | Compound No. | Chemical name | Status |
|---|---|---|---|
| 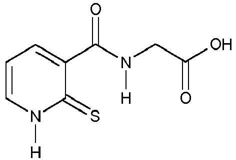 | 1 | N-[(2-thioxo-1,2-dihydropyridin-3-yl)carbonyl]glycine | Novel product |
| 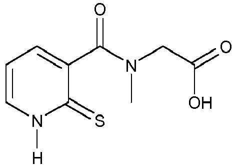 | 2 | N-methyl-N-[(2-thioxo-1,2-dihydropyridin-3-yl)carbonyl]glycine | Novel product |
| 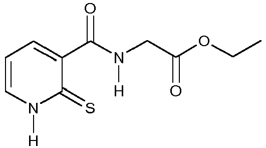 | 3 | Ethyl N-[(2-thioxo-1,2-dihydropyridin-3-yl)carbonyl]glycinate | Compound a) |
| 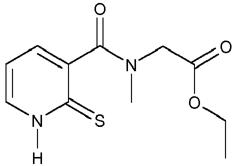 | 4 | Ethyl N-methyl-N-[(2-thioxo-1,2-dihydropyridin-3-yl)carbonyl]glycinate | Novel product |

and also the tautomers thereof, the salts thereof, the solvates thereof and the optical isomers thereof, and the racemates thereof, alone or as a mixture.

Among these compounds, compounds 1 and 2, and also the salts thereof, the solvates thereof, the isomers thereof and the racemates thereof, taken alone or as a mixture, are more particularly preferred.

The compounds of formula (I) can be obtained, in a known manner, by reacting 2-mercaptonicotinic acid and an amine of formula (V): 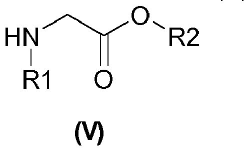 R1 and R2 having the meanings described above, in particular in the presence of a coupling agent or of a base such as carbonyldiimidazole.

The compounds of formulae (I) and (II) can also be obtained, in a known manner, by reacting 2-mercaptonicotinic acid or 2-chloronicotinic acid with an amine of formula (V) (R1 and R2 having the meanings described above), after having activated the carboxylic acid in the form of formula (W): 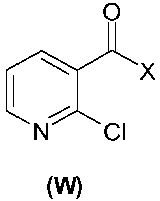 where X forms an acid halide, a mixed anhydride, a carbamimidate or an acylphosphonate by activation of the carboxylic group of the 2-chloronicotinic acid in the presence of an agent for activating carboxylic acids according to the conventional methods for activating acids (described for example in Comprehensive Organic Transformation by R. Larock, published by Wiley VCH, in the chapter Interconversion of nitriles, carboxylic acids and derivatives). Use is preferably made of an agent for activating carboxylic acids which makes it possible to form an acid chloride (for example by using thionyl or oxalyl chloride, or 1-chloro-N,N,2-trimethyl-1-propenamine) or to form a mixed anhydride (using alkyl or aryl chloroformates) or using carbodiimides or diethyl cyanophosphate to form carbamimidates or acylphosphonates (Phosphorus in organic synthesis - XI, Amino acids and peptides - XXI, Reaction of diethyl phosphorocyanidate with carboxylic acids. A new synthesis of carboxylic esters and amides, Tetrahedron, 32, 1976, 2211-2217).

When 2-chloronicotinic acid is used as starting reagent, the chloroamide of formula (Y) obtained is then used in an exchange reaction between the chlorine and the sulfur by means of reagents such as NaSH, thiourea, the Bunte salt, sodium thiosulfate or thioacetic acid (in a basic medium).

According to the overall synthesis scheme below: 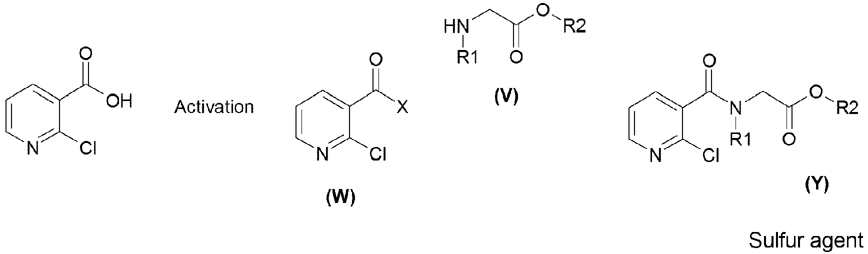 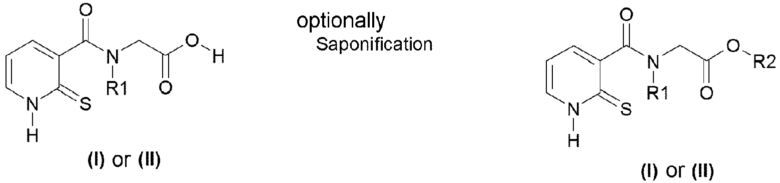 where R1, R2 and X have the same meanings as those previously defined.

In addition, when the final compounds (I) and (II) have a free carboxylic acid on the R2 radicals, said compounds can be obtained either from the corresponding amino acids or by saponification of the corresponding esters using inorganic bases such as, for example, NaOH or LiOH in the presence of protic or aprotic solvents such as, for example, ethanol or tetrahydrofuran or water, at temperatures ranging between zero and 100°C. The salts obtained can then be acidified with a mineral or organic acid such as, for example, hydrochloric acid or citric acid.

The compounds of formulae (I) and/or (II) according to the invention have a quite particular application in the cosmetics field.

The composition used according to the invention comprises a compound of formulae (I) and/or (II) as described above, in a physiologically acceptable medium.

The compound (I) and/or (II) may be present in the composition used according to the invention in an amount which may be between 0.01% and 10% by weight, preferably between 0.1% and 5% by weight, in particular from 0.5% to 3% by weight, relative to the total weight of the composition.

The term "physiologically acceptable medium" is intended to mean a medium that is compatible with human keratin materials such as the skin of the body or of the face, the lips, the mucous membranes, the eyelashes, the nails, the scalp and/or the hair.

The composition used according to the invention may then comprise any adjuvant commonly used in the cosmetics field. Mention may be made in particular of water; organic solvents, in particular C₁-C₆, more preferentially C₂-C₆, alcohols and C₂-C₁₀ carboxylic acid esters; oils, in particular hydrocarbon-based oils and/or silicone oils, of mineral, animal and/or plant origin; waxes, pigments, fillers, dyes, surfactants, emulsifiers; cosmetic or dermatological active agents, UV-screening agents, polymers, hydrophilic or lipophilic gelling agents, thickeners, preservatives, fragrances, bactericides, ceramides, odour absorbers and antioxidants.

These optional cosmetic adjuvants may be present in the composition in a proportion of from 0.001% to 80% by weight and in particular from 0.1% to 40% by weight relative to the total weight of the composition. In any case, these adjuvants, and the proportions thereof, will be chosen by those skilled in the art such that the advantageous properties of the compounds according to the invention are not, or are not substantially, adversely affected by the envisaged addition.

As active agents, it will be advantageous to introduce into the composition used according to the invention at least one compound chosen from: desquamating agents; soothing agents, organic or inorganic photo protective agents, moisturizers; depigmenting or propigmenting agents; anti-glycation agents; NO-synthase inhibitors; agents for stimulating the synthesis of dermal or epidermal macromolecules and/or for preventing their degradation; agents for stimulating fibroblast and/or keratinocyte proliferation or for stimulating keratinocyte differentiation; muscle relaxants and/or dermo-decontracting agents; tensioning agents; anti-pollution agents and/or free-radical scavengers; agents acting on the capillary circulation; agents acting on the energy metabolism of cells; and mixtures thereof.

Examples of such additional compounds are: retinol and derivatives thereof, such as retinyl palmitate; ascorbic acid and derivatives thereof, such as magnesium ascorbyl phosphate and ascorbyl glucoside; tocopherol and derivatives thereof, such as tocopheryl acetate; nicotinic acid and precursors thereof, such as nicotinamide; ubiquinone; glutathione and precursors thereof, such as L-2-oxothiazolidine-4-carboxylic acid; plant extracts, and in particular plant proteins and hydrolyzates thereof, and also phytohormones; marine extracts, such as algal extracts; bacterial extracts; sapogenins such as diosgenin and extracts of Wild Yam containing same; ceramides; hydroxy acids, such as salicylic acid and 5-n-octanoylsalicylic acid; resveratrol; oligopeptides and pseudodipeptides and acylated derivatives thereof; manganese salts and magnesium salts, in particular gluconates; and mixtures thereof.

The term "desquamating agent" is intended to mean any compound capable of acting:
- either directly on desquamation by promoting exfoliation, such as β-hydroxy acids, in particular salicylic acid and derivatives thereof (including 5-n-octanoylsalicylic acid); α-hydroxy acids, such as glycolic acid, citric acid, lactic acid, tartaric acid, malic acid or mandelic acid; urea; gentisic acid; oligofucoses; cinnamic acid; extract of *Saphora japonica;* resveratrol;
- or on the enzymes involved in the desquamation or degradation of corneodesmosomes, glycosidases, stratum corneum chymotryptic enzyme (SCCE) or other proteases (trypsin, chymotrypsin-like). Mention may be made of mineral salt chelating agents: EDTA; N-acyl-N,N',N'-ethylenediaminetriacetic acid; aminosulfonic compounds and in particular (N-2-hydroxyethylpiperazine-N-2-ethane)sulfonic acid (HEPES); 2-oxothiazolidine-4-carboxylic acid (procysteine) derivatives; derivatives of alpha-amino acids of glycine type (as described in EP 0 852 949, and also sodium methyl glycine diacetate sold by BASF under the trade name Trilon M); honey; sugar derivatives such as O-octanoyl-6-D-maltose and N-acetylglucosamine.

The desquamating agents are generally present in the composition according to the invention in proportions ranging from 0.01% to 15% by weight, preferably ranging from 0.1% to 10% by weight relative to the total weight of the composition.

As soothing agents that can be used in the composition according to the invention, mention may be made of pentacyclic triterpenes and extracts from plants (for example *Glycyrrhiza glabra*) containing the same, for instance β-glycyrrhetinic acid and salts and/or derivatives thereof (glycyrrhetinic acid monoglucuronide, stearyl glycyrrhetinate, 3-stearoyloxyglycyrrhetic acid), ursolic acid and salts thereof, oleanolic acid and salts thereof, betulinic acid and salts thereof, an extract of *Paeonia suffruticosa* and/or *lactiflora,* salicylic acid salts and in particular zinc salicylate, phycosaccharides from the company Codif, an extract of *Laminaria saccharina,* canola oil, bisabolol and extracts of camomile, allantoin, Sepivital EPC (phosphoric diester of vitamins E and C) from SEPPIC, omega-3 unsaturated oils such as musk rose oil, blackcurrant oil, ecchium oil, fish oil, plankton extracts, capryloylglycine, Seppicalm VG (sodium palmitoylproline and *Nymphea alba*) from SEPPIC, an extract of Pygeum, an extract of *Boswellia serrata,* an extract of *Centipeda cunninghami,* an extract of *Helianthus annuus,* an extract of *Linum usitatissimum,* tocotrienols, extracts of *Cola nitida,* piperonal, an extract of clove, an extract of *Epilobium angustifolium, Aloe vera,* an extract of *Bacopa moniera,* phytosterols, cortisone, hydrocortisone, indomethacin and betamethasone.

The soothing agents are generally present in the composition used according to the invention in proportions ranging from 0.01% to 15% by weight, preferably ranging from 0.1% to 10% by weight relative to the total weight of the composition.

The organic photo protective agents are in particular chosen from anthranilates; cinnamic derivatives; dibenzoylmethane derivatives; salicylic derivatives, camphor derivatives; triazine derivatives such as those described in patent applications US 4 367 390, EP 863 145, EP 517 104, EP 570 838, EP 796 851, EP 775 698, EP 878 469, EP 933 376, EP 507 691, EP 507 692, EP 790 243 and EP 944 624; benzophenone derivatives; β, β-diphenylacrylate derivatives; benzotriazole derivatives; benzalmalonate derivatives; benzimidazole derivatives; imidazolines; bis-benzazolyl derivatives as described in patents EP 669 323 and US 2 463 264; p-aminobenzoic acid (PABA) derivatives; methylenebis(hydroxyphenylbenzotriazole) derivatives as described in applications US 5 237 071, US 5 166 355, GB 2 303 549, DE 197 26 184 and EP 893 119; screening polymers and screening silicones such as those described in particular in application WO 93/04665; α-alkylstyrene-based dimers such as those described in patent application DE 198 55 649.

The inorganic photo protective agents can in particular be chosen from coated or uncoated metal oxide pigments or nanopigments (mean size of the primary particles generally between 5 nm and 100 nm, preferably between 10 nm and 50 nm), for instance titanium oxide (amorphous or crystallized in rutile and/or anatase form), iron oxide, zinc oxide, zirconium oxide or cerium oxide nanopigments, which are all well-known UV-photoprotective agents. Conventional coating agents are, moreover, alumina and/or aluminium stearate. Such coated or uncoated metal oxide nanopigments are described in particular in patent applications EP-518 772 and EP-518 773.

The photo protective agents are generally present in the composition used according to the invention in proportions ranging from 0.1% to 20% by weight, preferably ranging from 0.2% to 15% by weight relative to the total weight of the composition.

The composition used according to the invention may be in any galenical form normally used in the cosmetics field, and in particular in the form of an aqueous or aqueous-alcoholic solution, optionally gelled, a dispersion of the lotion type, optionally a two-phase dispersion, an oil-in-water or water-in-oil or multiple (W/O/W or O/W/O for example) emulsion, an aqueous gel, a dispersion of oil in an aqueous phase by means of spherules, these spherules possibly being polymer nanoparticles such as nanospheres and nanocapsules, or, better still, lipid vesicles of ionic and/or nonionic type; aqueous or oily gel. These compositions are prepared according to the usual methods. A composition in the form of an emulsion, in particular an oil-in-water emulsion, is preferably used according to this invention.

The composition used according to the invention may constitute a skincare composition, and in particular a cleansing, protecting, treating or care cream for the face, the hands, the feet, the major anatomical folds or the body (for example day creams, night creams, makeup-removing creams, foundation creams or anti-sun creams); a fluid foundation, a makeup-removing milk, a protective or care body milk or an anti-sun milk; a skincare lotion, gel or mousse, such as a cleansing lotion.

The invention is illustrated in greater detail by the following non-limiting examples.

### Example 1: Synthesis of compound 3 - ethyl N-[(2-thioxo-1,2-dihydropyridin-3-yl)carbonyl]glycinate

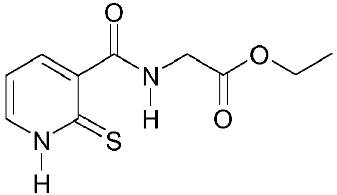

### Route 1: via 2-mercatonicotinic acid

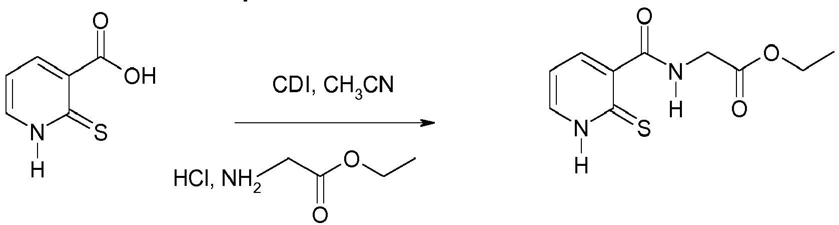

In a round-bottomed flask, thionicotinic acid is introduced into acetonitrile, followed by carbonyldiimidazole (CDI). The mixture is refluxed for 1 hour. The mixture is returned to ambient temperature and ethyl glycinate hydrochloride is added and then the heating is recommenced at 60°C for 3 hours and then overnight at ambient temperature.

After the overnight period, the reaction medium is evaporated. The residue is taken up in dichloromethane and washed twice with 2 N HCl. The organic phase is dried over anhydrous sodium sulfate and then evaporated and purified on silica, 97/3 dichloromethane/methanol.

The fractions are evaporated.

A light-yellow-coloured powder is obtained: Yield: 37%.

### Route 2: via 2-chloronicotinic acid

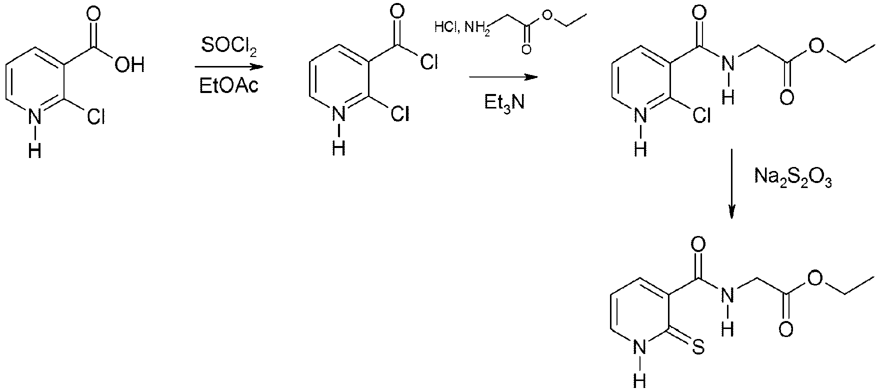

In a three-necked flask, 2-chloronicotinic acid (250.0 g, 1.587 mol) is introduced into ethyl acetate (500 ml) and SOCl₂ (210.0 g, 1.761 mol), dropwise. The mixture is refluxed until complete conversion is obtained. The medium is cooled to ambient temperature and diluted with ethyl acetate (375 ml). A solution of ethyl glycinate hydrochloride (265.8 g, 1.904 mol) diluted in water (400 ml) and triethylamine (393.5 g, 3.88 mol) are then added. The mixture is stirred for 3 to 4 h and the ethyl acetate is removed under vacuum. The aqueous solution obtained is diluted with water (1250 ml) and acidified with 3 N HCI (25 ml). Sodium thiosulfate (1379 g, 5.555 mol) is charged to the resulting solution and the mixture is refluxed for 6 h. After cooling to 10°C, the yellow solid is filtered off and washed with water (3×750 ml).

The crude product is taken up with carbon black in a 75/25 ethanol/water mixture. The carbon black is filtered off under hot conditions and the ethanol is evaporated off. The product is cooled to ambient temperature, filtered and dried under vacuum. A light-yellow-coloured powder is obtained: Yield: 88%.

The ¹H NMR and mass spectra are in accordance with the structure.

Melting point: 151°C (capillary tube)

### Example 2: Synthesis of compound 1

### N-[(2-thioxo-1,2-dihydropyridin-3-yl)carbonyl]glycine from compound 3

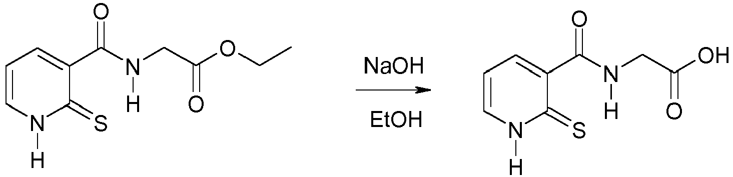

Ethyl N-[(2-thioxo-1,2-dihydropyridin-3-yl)carbonyl]glycinate (48 g, 0.317mol), 95% EtOH (48ml) are introduced into a three-necked flask and cooled to 10°C. A solution of NaOH (16g) in 144 ml of water is added dropwise. The organic solvent is evaporated off and the pH is adjusted to 3-4. The resulting product is cooled to 0-10°C and filtered. The product is washed with water and dried under vacuum.

A light-yellow-coloured powder is obtained: Yield: 96%.

The ¹H NMR and mass spectra are in accordance with the structure.

Melting point: 241.0-241.8°C (capillary tube)

### Example 3: Synthesis of compound 4 - ethyl N-methyl-N-[(2-thioxo-1,2-dihydropyridin-3-yl)carbonyl]glycinate

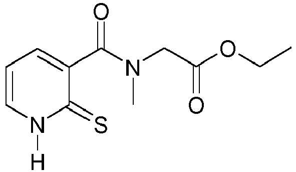

### Route 1: via 2-mercaptonicotinic acid

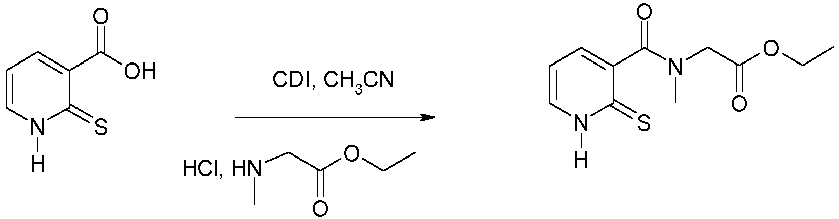

In a round-bottomed flask, thionicotinic acid is introduced into acetonitrile, followed by carbonyldiimidazole (CDI). The mixture is refluxed for 1 hour. The mixture is returned to ambient temperature and ethyl sarcosinate hydrochloride is added and then the heating is recommenced at 60°C for 3 hours and then overnight at ambient temperature.

After the overnight period, the reaction medium is evaporated. The residue is purified on silica, 20/80 ethyl acetate/heptane.

The fractions are evaporated and then the product is taken up in dichloromethane and precipitated from isopropyl ether.

A light-yellow-coloured powder is obtained: Yield: 22%.

### Route 2: via 2-chloronicotinic acid

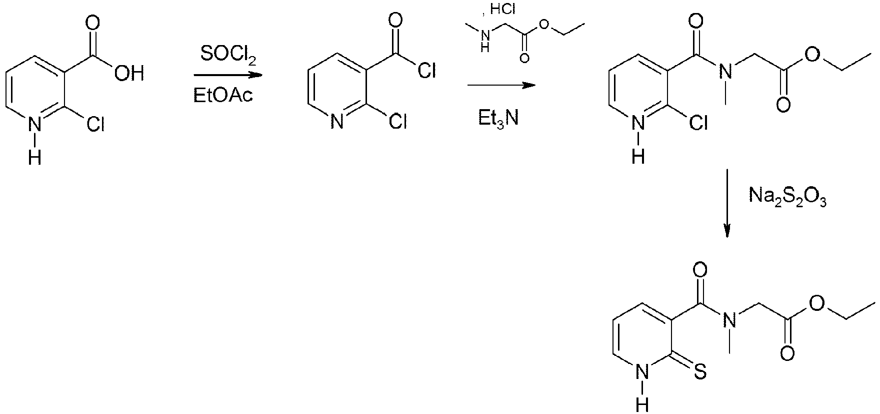

In a three-necked flask, 2-chloronicotinic acid (300.0 g, 1.904 mol) is introduced into ethyl acetate (600 ml) and SOCl₂ (249.0 g, 2.095 mol), dropwise. The mixture is refluxed until complete conversion is obtained. The medium is cooled to ambient temperature and diluted with ethyl acetate (450 ml). A solution of ethyl sarcosinate hydrochloride (351 g, 2.285 mol) diluted in water (480 ml) and triethylamine (481.8 g, 4.76 mol) are then added. The mixture is stirred for 3 to 4 h and the ethyl acetate is removed under vacuum. The aqueous solution obtained is diluted with water (1500 ml) and acidified with 3 N HCI (30 ml). Sodium thiosulfate (1796 g, 7.24 mol) is charged to the resulting solution and the mixture is refluxed until complete conversion is obtained. After cooling to 10°C, the yellow solid is filtered off and washed with water (3×900 ml).

The crude product is taken up with carbon black in a 75/25 ethanol/water mixture. The carbon black is filtered off under hot conditions and the ethanol is evaporated off. The product is cooled to ambient temperature, filtered and dried under vacuum. A light-yellow-coloured powder is obtained: Yield: 69%.

The ¹H NMR and mass spectra are in accordance with the structure.

Melting point: 175°C (capillary tube)

### Example 4: Synthesis of compound 2 from compound 4 N-methyl-N-[(2-thioxo-1,2-dihydropyridin-3-yl)carbonyl]glycine

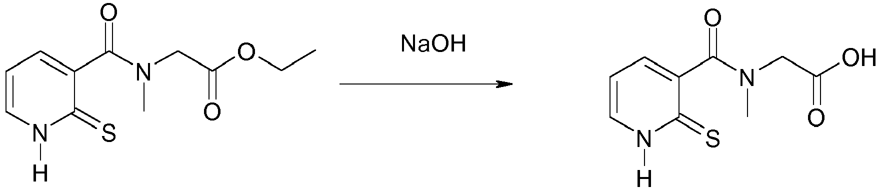

Ethyl N-methyl-N-[(2-thioxo-1,2-dihydropyridin-3-yl)carbonyl]glycinate (50 g, 0.317 mol), 95% EtOH (100 ml) and water (80 ml) are introduced into a three-necked flask. The mixture is cooled to 10°C. A solution of NaOH (23.6 g) in 70 ml of water is added dropwise. After complete conversion, the organic solvent is evaporated off and the pH is adjusted to 2-3. The resulting product is cooled to 0-10°C and filtered. The product is washed with water and dried under vacuum. A light-yellow-coloured powder is obtained: Yield: 45%.

The ¹H NMR and mass spectra are in accordance with the structure.

Melting point: 210.9-221.8°C (capillary tube)

### Example 5: Demonstration of the activity on constitutive melanogenesis

For the evaluations of the effect of prevention or of decrease of pigmentation of the skin and/or of lightening of the skin, the examples are carried out in the following way.

The measurement of the depigmenting activity (reduction of melanin production) of compounds of formula (I) was carried out by assaying normal human melanocytes in vitro as follows.

First of all, normal human melanocytes are cultured and dispensed into 384 wells. After 24 hours, the culture medium was replaced with a medium containing compounds of formula (I) to be evaluated. The cells were incubated for 72 hours before measuring the final optical density, which measures the amount of melanin produced by the melanocytes. A dose-effect is performed using a wide concentration range of the compounds evaluated. Thus, by making the concentrations and the melanin measurements correspond, it is possible to determine an IC50 in µM: concentration at which 50% decrease in melanin synthesis is achieved. The highest concentration used in the test is 200 µM.

The compounds of formula (I) showed a strong depigmenting effect.

| Compound No. | IC50 (µM) |
|---|---|
| 1 | 11.4 |
| 2 | 5.27 |
| 3 | 36.8 |
| 4 | 67.6 |

In another campaign, compound 3 was compared with the closest compound of the prior art described in patent FR 2 968 661:

| Compound No. | IC50 (µM) |
|---|---|
| 3 | 20.8 |
| 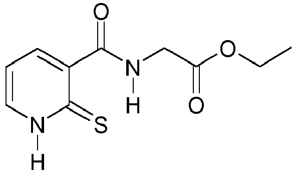 | |
| 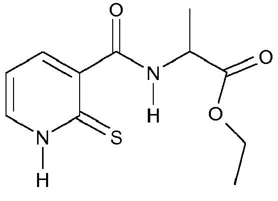 | 37.4 |
| CAS 1379867-59-0 | |

The compounds of the invention have an activity on melanogenesis reduction that is greater than that of the compound (CAS 1379867-59-0) outside the invention.

### Example 6: Cosmetic composition

A skin depigmenting composition is prepared, comprising (in grams):

| | |
|---|---|
| Compound No. 4 | 2 g |
| PEG 400 | 68 g |
| Ethanol | 30 g |

The composition applied to the skin makes it possible to cause brown spots to become less marked.

### Example 7: gel

A skin depigmenting gel is prepared, comprising (% by weight):

| | |
|---|---|
| Compound No. 1 | 0.5% |
| Carbomer (Carbopol 981 from Lubrizol) | 1% |
| preservative | qs |
| water | qs 100% |

The composition applied to the skin makes it possible to cause brown spots to become less marked.

### Example 8 :

### Test on pigmented reconstructed epidermis samples

Compositions comprising 300 µM of compound 1, 2, 3 or 4 in DMSO were applied to samples of pigmented reconstructed epidermis (cf. EP 1 878 790). The control is DMSO. The melanin was quantified by image analysis on histological slices after staining with Fontana Masson dye. Each sample of coloured epidermis is photographed over its entire length using a camera connected to a microscope. The melanin is thresholded and the number of melanin pixels is measured in each field using automated image analysis software. A non-parametric statistical test is performed in order to determine the significance of the measurements (Mann-Whitney test).

The pigmented reconstructed epidermis standard study model was published by:
Regnier M, Duval C, Galey JB, Philippe M, Lagrange A, Tuloup R, Schmidt R, Cellular and Molecular Biology, 1999, 45, 7, 969-980: "Keratinocyte-Melanocyte co-cultures and pigmented reconstructed human epidermis: models to study modulation of melanogenesis".

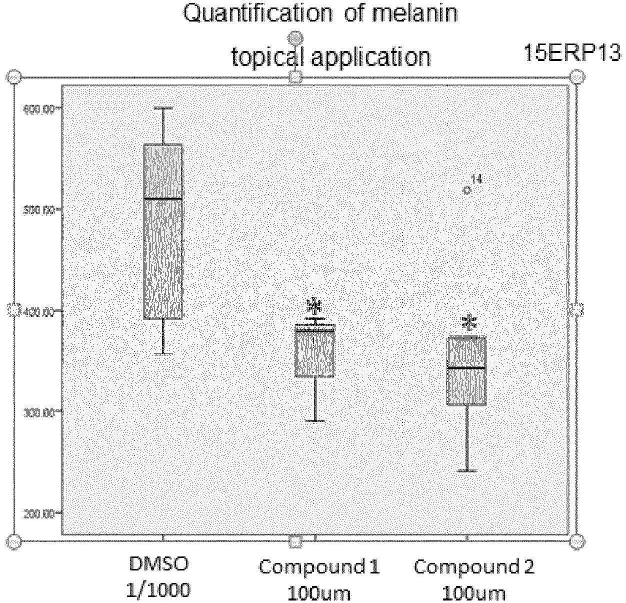

Significant depigmenting activity was evaluated at 100 µM for compounds 1 and 2.(pValue < 0.05: significant depigmenting activity).

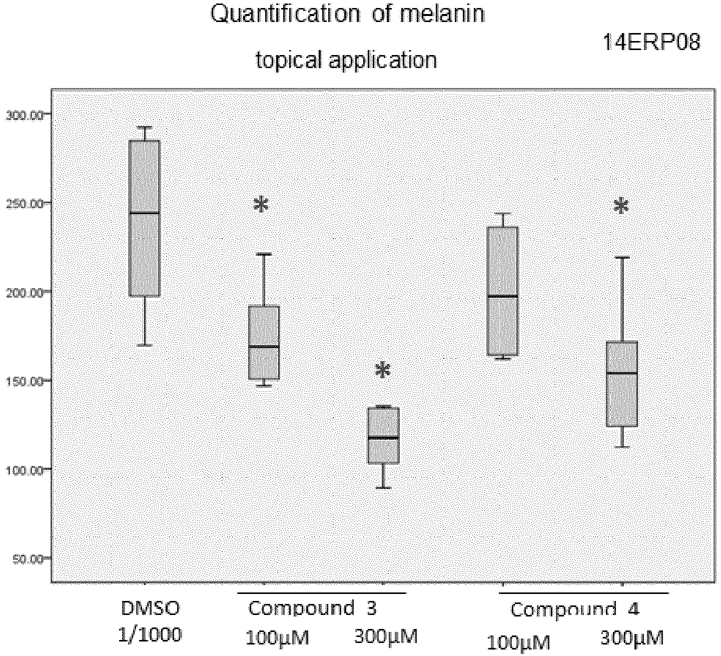

Significant depigmenting activity was evaluated at 300 µM for compounds 3 and 4.(pValue < 0.05: significant depigmenting activity).

## Claims

1. Non-therapeutic cosmetic process for depigmenting, lightening and/or bleaching keratin materials, comprising the application of a cosmetic composition comprising, in a physiologically acceptable medium, at least one compound of formula (I): 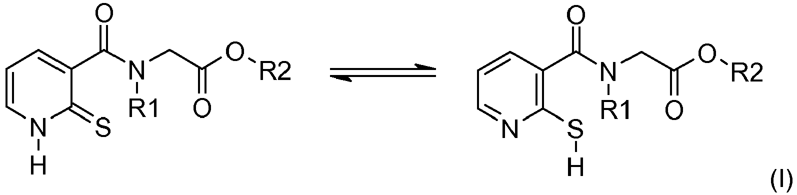 in which:
R1 denotes a radical chosen from:
a) a hydrogen atom;
b) a saturated linear C₁-C₆ alkyl group;
R2 denotes a radical chosen from:
a) a hydrogen atom;
b) a saturated linear C₁-C₁₀ alkyl group;
c) a saturated branched C₃-C₁₀ alkyl group;
d) a C₁-C₆ phenylalkyl group such as benzyl,
and also the tautomers thereof, the salts thereof, the solvates thereof and the optical isomers thereof, and the racemates thereof, alone or as a mixture.

2. Process according to Claim 1, in which:
R1 denotes a radical chosen from:
a) a hydrogen atom;
b) a saturated linear C₁-C₄ alkyl radical and preferably methyl;
R2 denotes a radical chosen from:
a) a hydrogen atom;
b) a saturated linear C₁-C₆ alkyl group;
c) a saturated branched C₃-C₆ alkyl group.

3. Process according to either of the preceding claims, in which:
R1 denotes a radical chosen from:
a) a hydrogen atom;
b) a methyl radical;
R2 denotes a radical chosen from:
a) a hydrogen atom;
b) a saturated linear C₁-C₄ alkyl group, preferably ethyl;
c) a saturated branched C₃-C₄ alkyl group, preferably isopropyl and isobutyl.

4. Process according to one of the preceding claims, in which the compounds of formula (I) are chosen from:
| Structure | Compound No. | Chemical name |
|---|---|---|
| 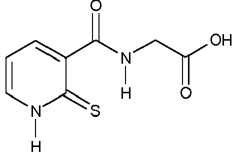 | 1 | N-[(2-thioxo-1,2-dihydropyridin-3-yl)carbonyl]glycine |
| 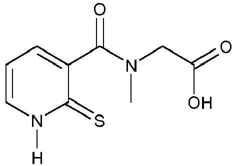 | 2 | N-methyl-N-[(2-thioxo-1,2-dihydropyridin-3-yl)carbonyl]glycine |
| 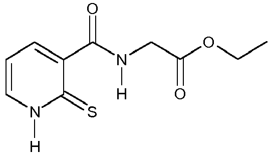 | 3 | Ethyl N-[(2-thioxo-1,2-dihydropyridin-3-yl)carbonyl]glycinate |
| 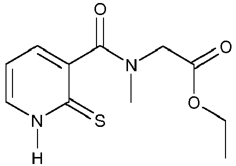 | 4 | Ethyl N-methyl-N-[(2-thioxo-1,2-dihydropyridin-3-yl)carbonyl]glycinate |
and also the tautomers thereof, the salts thereof, the solvates thereof and the optical isomers thereof, and the racemates thereof, alone or as a mixture.

5. Process according to Claim 1, in which the compound of formula (I) is chosen from the following compounds:
| Structure | Compound No. | | Chemical name |
|---|---|---|---|
| 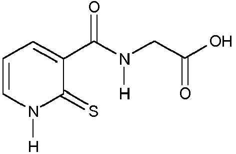 | 1 | | N-[(2-thioxo-1,2-dihydropyridin-3-yl)carbonyl] glycine |
| 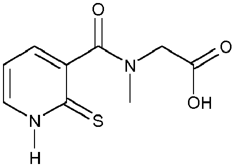 | 2 | | N-methyl-N-[(2-thioxo-1,2-dihydropyridin-3-yl)carbonyl]glycine |

6. Process according to one of the preceding claims, in which the compound of formula (I) is present, alone or as a mixture, in the composition, in an amount between 0.01% and 10% by weight, preferably between 0.1% and 5% by weight and in particular between 0.5% and 3% by weight, relative to the total weight of the composition.

7. Process according to one of the preceding claims, in which the composition comprises at least one adjuvant chosen from the group made up of: water; organic solvents, in particular C₁-C₆ alcohols and C₂-C₁₀ carboxylic acid esters; carbon-based and/or silicone oils, of mineral, animal and/or plant origin; waxes, pigments, fillers, dyes, surfactants, emulsifiers, co-emulsifiers; cosmetic or dermatological active agents, UV screening agents, polymers, hydrophilic or lipophilic gelling agents, thickeners, preservatives, fragrances, bactericides, ceramides, odour absorbers, antioxidants.

8. Process according to one of the preceding claims, in which the composition comprises at least one active agent chosen from: desquamating agents; soothing agents, organic or inorganic photo protective agents, moisturizers; depigmenting or propigmenting agents; anti-glycation agents; NO-synthase inhibitors; agents for stimulating the synthesis of dermal or epidermal macromolecules and/or for preventing their degradation; agents for stimulating fibroblast and/or keratinocyte proliferation or for stimulating keratinocyte differentiation; muscle relaxants and/or dermo-decontracting agents; tensioning agents; anti-pollution agents and/or free-radical scavengers; agents acting on the capillary circulation; agents acting on the energy metabolism of cells; and mixtures thereof.

9. Non-therapeutic cosmetic use of a compound of formula (I) as defined according to any one of Claims 1 to 6, as an agent for bleaching, lightening and/or depigmenting keratin materials.

10. Compounds of formula (II): 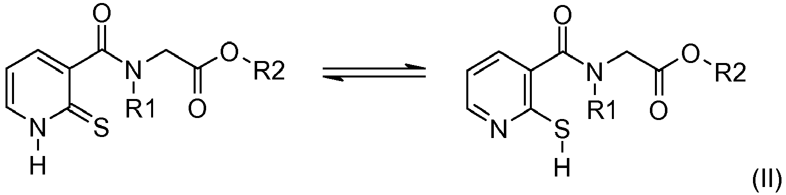 in which:
R1 denotes a radical chosen from:
a) a hydrogen atom;
b) a saturated linear C₁-C₆ alkyl group;
R2 denotes a radical chosen from:
a) a hydrogen atom;
b) a saturated linear C₁-C₁₀ alkyl group;
c) a saturated branched C₃-C₁₀ alkyl group;
d) a C₁-C₆ phenylalkyl group such as benzyl,
and also the tautomers thereof, the salts thereof, the solvates thereof and the optical isomers thereof, and the racemates thereof, alone or as a mixture,
with the exception of the following two compounds (a) and (b) and of the tautomers thereof:

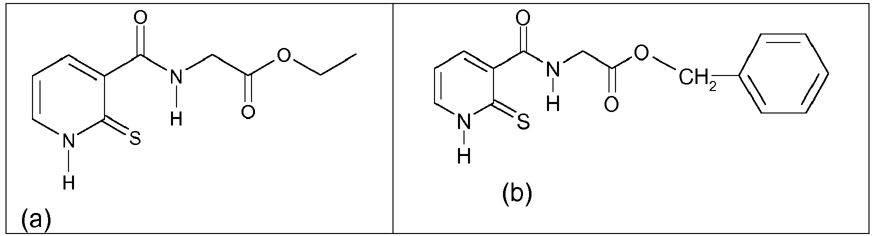

11. Compounds of formula (II) according to the preceding claim, for which:
R1 denotes a radical chosen from:
a) a hydrogen atom;
b) a saturated linear C₁-C₄ alkyl radical and preferably methyl;
R2 denotes a radical chosen from:
a) a hydrogen atom;
b) a saturated linear C₁-C₆ alkyl group;
c) a saturated branched C₃-C₆ alkyl group,
and also the tautomers thereof, the salts thereof, the solvates thereof and the optical isomers thereof, and the racemates thereof, alone or as a mixture,
with the exception of the following compound (a) and of the tautomer thereof:

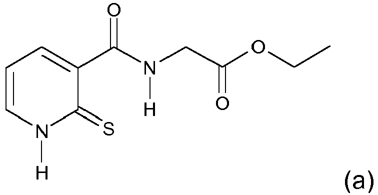

12. Compounds of formula (II) according to either of Claim 10 and 11, chosen from:
| Structure | Compound No. | | Chemical name |
|---|---|---|---|
| 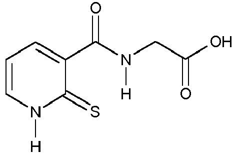 | 1 | | N-[(2-thioxo-1,2-dihydropyridin-3-yl)carbonyl]glycine |
| 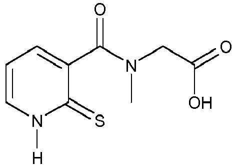 | 2 | | N-methyl-N-[(2-thioxo-1,2-dihydropyridin-3-yl)carbonyl]glycine |
| 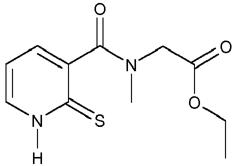 | 4 | | ethyl N-methyl-N-[(2-thioxo-1,2-dihydropyridin-3-yl)carbonyl]glycinate |
and also the tautomers thereof, the salts thereof, the solvates thereof and the optical isomers thereof, and the racemates thereof, alone or as a mixture.

13. Composition, in particular cosmetic composition, comprising at least one compound of formula (II) according to one of Claims 10 to 12.

14. Composition according to the preceding claim, **characterized in that** said compound of formula (II) is present in an amount of between 0.01% and 10% by weight, preferably between 0.1% and 5% by weight and in particular between 0.5% and 3% by weight, relative to the total weight of the composition.

15. Process for preparing compounds of formulae (I) and (II) as defined in any one of Claims 1 to 12, according to the following scheme: 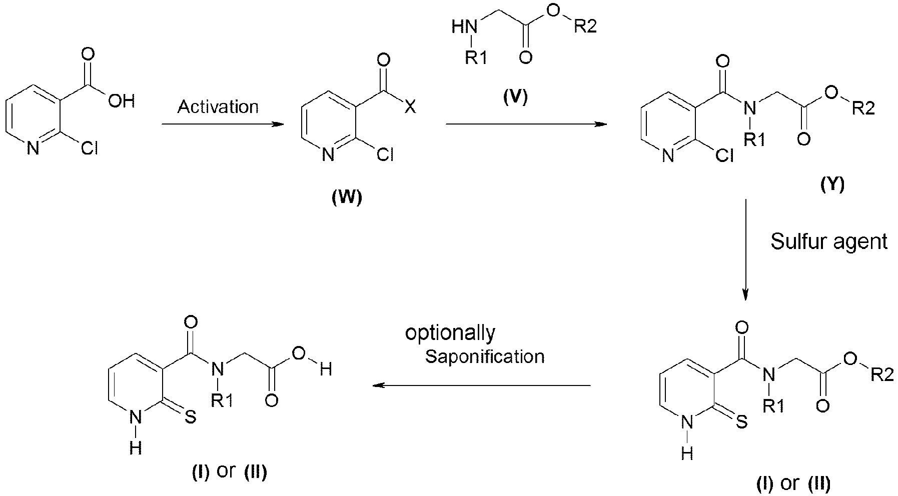 where R1, R2 and X have the same meanings as those previously defined.

16. Preparation process according to the preceding claim, which comprises the following steps:
(i) the step of preparing a compound of formula (W) 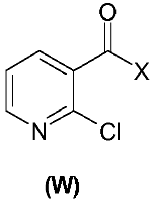 where X forms an acid halide, a mixed anhydride, a carbamimidate or an acylphosphonate by activation of the carboxylic group of the 2-chloronicotinic acid in the presence of an agent for activating carboxylic acids according to the conventional methods for activating acids; then
ii) the step of reacting the compound (W) with an amine of formula (V) 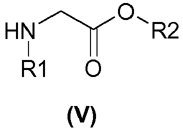 where R1 and R2 have the same meanings as those described in Claim 1 so as to form the compound of formula (Y); 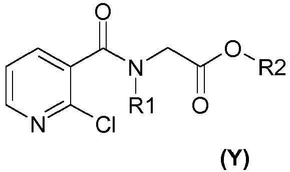 then
iii) the step of exchange between the chlorine and the sulfur by means of reagents such as sodium disulfite, thiourea, sodium thiosulfate or thioacetic acid so as to form a compound of formulae (I) and (II), then optionally
iv) for the compounds of formulae (I) and (II) with the R2 radical representing hydrogen, said compounds can be obtained either directly or by means of an additional step of saponification of the corresponding esters using inorganic bases following acidification.

## Patentansprüche

1. Nichttherapeutisches kosmetisches Verfahren zur Depigmentierung, Aufhellung und/oder Bleichung von Keratinmaterialien, bei dem man ein kosmetische Zusammensetzung aufbringt, die in einem physiologisch unbedenklichen Medium mindestens eine Verbindung der Formel (I) 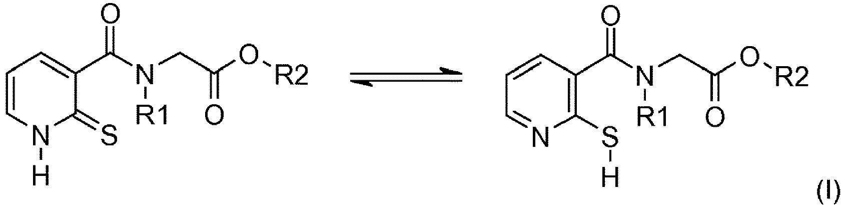 in der:
R1 für einen Rest, der aus
a) einem Wasserstoffatom,
b) einer gesättigten linearen C₁-C₆-Alkylgruppe ausgewählt ist, steht;
R2 für einen Rest, der aus
a) einem Wasserstoffatom,
b) einer gesättigten linearen C₁-C₁₀-Alkylgruppe,
c) einer gesättigten verzweigten C₃-C₁₀-Alkylgruppe,
d) einer C₁-C₆-Phenylalkylgruppe wie Benzyl ausgewählt ist, steht;
sowie die Tautomere davon, die Salze davon, die Solvate davon und die optischen Isomere davon und die Racemate davon, alleine oder als Gemisch, umfasst.

2. Verfahren nach Anspruch 1, bei dem:
R1 für einen Rest, der aus
a) einem Wasserstoffatom,
b) einem gesättigten linearen C₁-C₄-Alkylrest und vorzugsweise Methyl
ausgewählt ist, steht;
R2 für einen Rest, der aus
a) einem Wasserstoffatom,
b) einer gesättigten linearen C₁-C₆-Alkylgruppe,
c) einer gesättigten verzweigten C₃-C₆-Alkylgruppe
ausgewählt ist, steht.

3. Verfahren nach einem der vorhergehenden Ansprüche, bei dem:
R1 für einen Rest, der aus
a) einem Wasserstoffatom,
b) einem Methylrest
ausgewählt ist, steht;
R2 für einen Rest, der aus
a) einem Wasserstoffatom,
b) einer gesättigten linearen C₁-C₄-Alkylgruppe, vorzugsweise Ethyl,
c) einer gesättigten verzweigten C₃-C₄-Alkylgruppe, vorzugsweise Isopropyl und Isobutyl,
ausgewählt ist, steht.

4. Verfahren nach einem der vorhergehenden Ansprüche, wobei die Verbindungen der Formel (I) ausgewählt sind aus:
| Struktur | Verbindung Nr. | Chemischer Name |
|---|---|---|
| 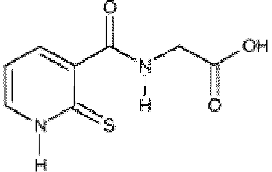 | 1 | N-[(2-Thioxo-1,2-dihydropyridin-3-yl)carbonyl]glycin |
| 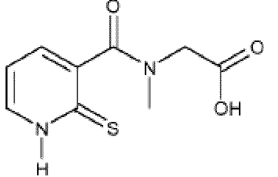 | 2 | N-Methyl-N-[(2-thioxo-1,2-dihydropyridin-3-yl)carbonyl]glycin |
| 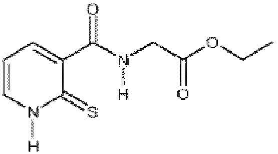 | 3 | N-[(2-Thioxo-1,2-dihydropyridin-3-yl)carbonyl]glycin-ethylester |
| 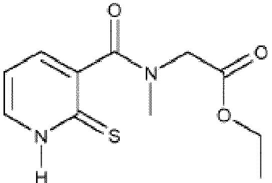 | 4 | N-Methyl-N-[(2-thioxo-1,2-dihydropyridin-3-yl)carbonyl]glycin-ethylester |
sowie den Tautomeren davon, den Salzen davon, den Solvaten davon und den optischen Isomeren davon und den Racematen davon, alleine oder als Gemisch.

5. Verfahren nach Anspruch 1, wobei die Verbindung der Formel (I) ausgewählt ist aus:
| Struktur | Verbindung Nr. | Chemischer Name |
|---|---|---|
| 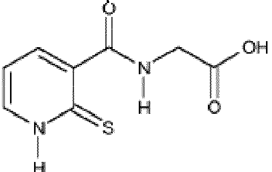 | 1 | N-[(2-Thioxo-1,2-dihydropyridin-3-yl)carbonyl]glycin |
| 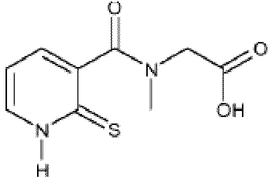 | 2 | N-Methyl-N-[(2-thioxo-1,2-dihydropyridin-3-yl)carbonyl]glycin |

6. Verfahren nach einem der vorhergehenden Ansprüche, bei dem die Verbindung der Formel (I), alleine oder als Gemisch, in der Zusammensetzung in einer Menge zwischen 0,01 und 10 Gew.-%, vorzugsweise zwischen 0,1 und 5 Gew.-% und insbesondere zwischen 0,5 und 3 Gew.-%, bezogen auf das Gesamtgewicht der Zusammensetzung, vorliegt.

7. Verfahren nach einem der vorhergehenden Ansprüche, bei dem die Zusammensetzung mindestens einen Hilfsstoff aus der Gruppe bestehend aus Wasser; organischen Lösungsmitteln, insbesondere C₁-C₆-Alkoholen und C₂-C₁₀-Carbonsäureestern; auf Kohlenstoff basierenden Ölen und/oder Silikonölen mineralischer, tierischer und/oder pflanzlicher Herkunft; Wachsen, Pigmenten, Füllstoffen, Farbstoffen, Tensiden, Emulgatoren, Coemulgatoren; kosmetischen oder dermatologischen Wirkstoffen, UV-Schutzmitteln, Polymeren, hydrophilen oder lipophilen Gelbildnern, Verdickungsmitteln, Konservierungsmitteln, Duftstoffen, Bakteriziden, Ceramiden, Geruchsabsorbern und Antioxidantien umfasst.

8. Verfahren nach einem der vorhergehenden Ansprüche, wobei die Zusammensetzung mindestens einen Wirkstoff umfasst, der aus Entschuppungsmitteln; beruhigend wirkenden Mitteln; organischen oder anorganischen Lichtschutzmitteln; Feuchtigkeitsmitteln; Depigmentierungsmitteln oder Propigmentierungsmitteln; Antiglykierungsmitteln; NO-Synthase-Inhibitoren; Mitteln zur Stimulierung der Synthese von dermalen oder epidermalen Makromolekülen und/oder zur Verhinderung ihrer Zersetzung; Mitteln zur Stimulierung der Proliferation von Fibroblasten und/oder Keratinozyten oder zur Stimulierung der Differenzierung von Keratinozyten; Muskelrelaxantien und/oder Hautdekontraktionsmitteln; Straffungsmitteln; Mitteln gegen Schadstoffe und/oder Radikalfängern; Mitteln, die auf die Kapillarzirkulation wirken; Mitteln, die auf den Energiestoffwechsel von Zellen wirken; und Mischungen davon ausgewählt ist.

9. Nichttherapeutische kosmetische Verwendung einer Verbindung der Formel (I) gemäß einem der Ansprüche 1 bis 6 als Mittel zur Bleichung, Aufhellung und/oder Depigmentierung von Keratinmaterialien.

10. Verbindungen der Formel (II) 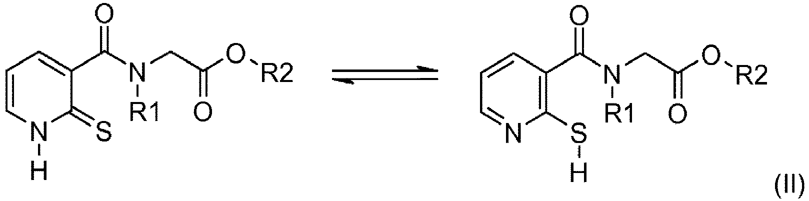 in der:
R1 für einen Rest, der aus
a) einem Wasserstoffatom,
b) einer gesättigten linearen C₁-C₆-Alkylgruppe ausgewählt ist, steht;
R2 für einen Rest, der aus
a) einem Wasserstoffatom,
b) einer gesättigten linearen C₁-C₁₀-Alkylgruppe,
c) einer gesättigten verzweigten C₃-C₁₀-Alkylgruppe,
d) einer C₁-C₆-Phenylalkylgruppe wie Benzyl ausgewählt ist, steht;
sowie die Tautomere davon, die Salze davon, die Solvate davon und die optischen Isomere davon und die Racemate davon, alleine oder als Gemisch,
mit Ausnahme der folgenden beiden Verbindungen (a) und (b) und der Tautomere davon:

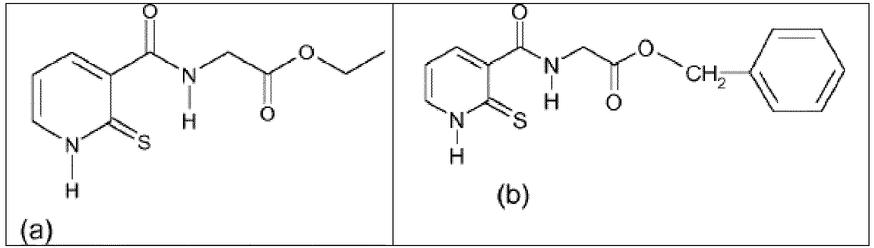

11. Verbindungen der Formel (II) nach dem vorhergehenden Anspruch, für die:
R1 für einen Rest, der aus
a) einem Wasserstoffatom,
b) einem gesättigten linearen C₁-C₄-Alkylrest und vorzugsweise Methyl
ausgewählt ist, steht;
R2 für einen Rest, der aus
a) einem Wasserstoffatom,
b) einer gesättigten linearen C₁-C₆-Alkylgruppe,
c) einer gesättigten verzweigten C₃-C₆-Alkylgruppe
ausgewählt ist, steht,
sowie die Tautomere davon, die Salze davon, die Solvate davon und die optischen Isomere davon und die Racemate davon, alleine oder als Gemisch,
mit Ausnahme der folgenden Verbindung (a) und des Tautomers davon:

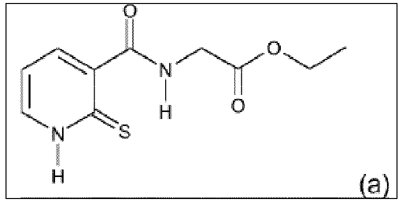

12. Verbindungen der Formel (II) nach Anspruch 10 oder 11, ausgewählt aus:
| Struktur | Verbindung Nr. | Chemischer Name |
|---|---|---|
| 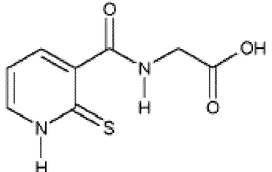 | 1 | N-[(2-Thioxo-1,2-dihydropyridin-3-yl)carbonyl]glycin |
| 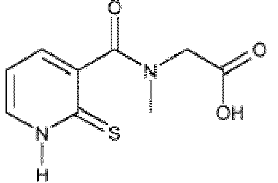 | 2 | N-Methyl-N-[(2-thioxo-1,2-dihydropyridin-3-yl)carbonyl]glycin |
| 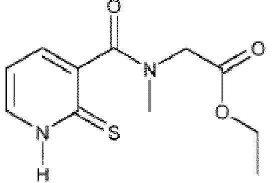 | 4 | N-Methyl-N-[(2-thioxo-1,2-dihydropyridin-3-yl)carbonyl]glycin-ethylester |
sowie die Tautomere davon, die Salze davon, die Solvate davon und die optischen Isomere davon und die Racemate davon, alleine oder als Gemisch.

13. Zusammensetzung, insbesondere kosmetische Zusammensetzung, die mindestens eine Verbindung der Formel (II) nach einem der Ansprüche 10 bis 12 umfasst.

14. Zusammensetzung nach dem vorhergehenden Anspruch, **dadurch gekennzeichnet, dass** die Verbindung der Formel (II) in einer Menge zwischen 0,01 und 10 Gew.-%, vorzugsweise zwischen 0,1 und 5 Gew.-% und insbesondere zwischen 0,5 und 3 Gew.-%, bezogen auf das Gesamtgewicht der Zusammensetzung, vorliegt.

15. Verfahren zur Herstellung von Verbindungen der Formeln (I) und (II) gemäß einem der Ansprüche 1 bis 12 gemäß dem folgenden Schema: 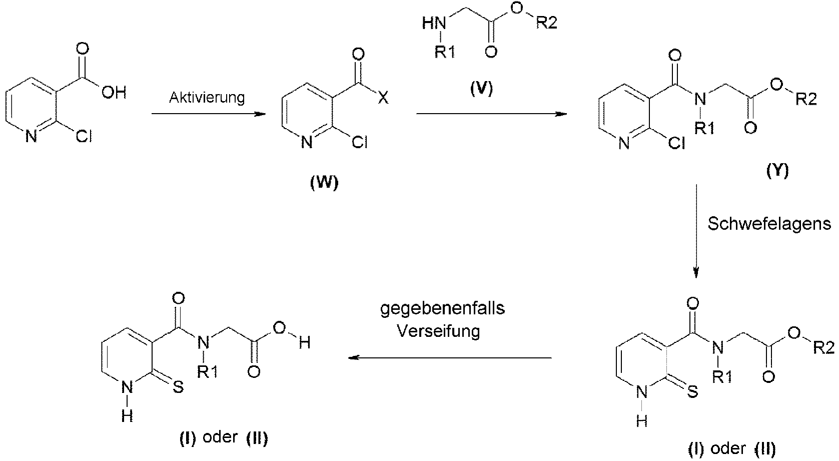 wobei R1, R2 und X die gleichen Bedeutungen wie oben definiert haben.

16. Herstellungsverfahren nach dem vorhergehenden Anspruch, das die folgenden Schritte umfasst:
(i) den Schritt der Herstellung einer Verbindung der Formel (W) 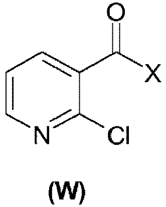 wobei X ein Säurehalogenid, ein gemischtes Anhydrid, ein Carbamimidat oder ein Acylphosphonat bildet, durch Aktivierung der Carboxylgruppe der 2-Chlornicotinsäure in Gegenwart eines Mittels zur Aktivierung von Carbonsäuren gemäß den herkömmlichen Methoden zur Aktivierung von Säuren; dann
ii) den Schritt der Umsetzung der Verbindung (W) mit einem Amin der Formel (V) 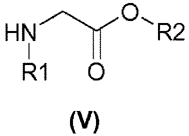 wobei R1 und R2 die gleichen Bedeutungen wie in Anspruch 1 beschrieben haben, zur Bildung der Verbindung der Formel (Y); 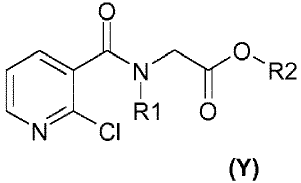 dann
iii) den Schritt des Austauschs zwischen dem Chlor und dem Schwefel mit Hilfe von Reagenzien wie Natriumdisulfit, Thioharnstoff, Natriumthiosulfat oder Thioessigsäure zur Bildung einer Verbindung der Formeln (I) und (II), dann gegebenenfalls
iv) für die Verbindungen der Formeln (I) und (II), in denen der R2-Rest für Wasserstoff steht, können die Verbindungen entweder direkt oder mit Hilfe eines zusätzlichen Schritts der Verseifung der entsprechenden Ester mit anorganischen Basen nach Ansäuerung erhalten werden.

## Revendications

1. Procédé cosmétique non thérapeutique destiné à la dépigmentation, l'éclaircissement et/ou la décoloration de matières kératiniques, comprenant l'application d'une composition cosmétique comprenant, dans un milieu physiologiquement acceptable, au moins un composé de formule (I) : 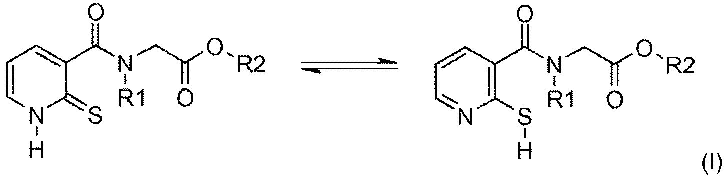 dans lequel
R1 désigne un radical choisi parmi :
a) un atome d'hydrogène ;
b) un groupement C₁-C₆-alkyle linéaire saturé ;
R2 désigne un radical choisi parmi :
a) un atome d'hydrogène ;
b) un groupement C₁-C₁₀-alkyle linéaire saturé ;
c) un groupement C₃-C₁₀-alkyle ramifié saturé ;
d) un groupement C₁-C₆-phénylalkyle tel que benzyle ;
ainsi que les tautomères de celui-ci, les sels de celui-ci, les solvates de celui-ci et les isomères optiques de celui-ci, et les racémates de celui-ci, seuls ou sous forme de mélange.

2. Procédé selon la revendication 1, dans lequel :
R1 désigne un radical choisi parmi :
a) un atome d'hydrogène ;
b) un radical C₁-C₄-alkyle linéaire saturé, et préférablement méthyle ;
R2 désigne un radical choisi parmi :
a) un atome d'hydrogène ;
b) un groupement C₁-C₆-alkyle linéaire saturé ;
c) un groupement C₃-C₆-alkyle ramifié saturé.

3. Procédé selon l'une ou l'autre parmi les revendications précédentes, dans lequel :
R1 désigne un radical choisi parmi :
a) un atome d'hydrogène ;
b) un radical méthyle ;
R2 désigne un radical choisi parmi :
a) un atome d'hydrogène ;
b) un groupement C₁-C₄-alkyle linéaire saturé, préférablement éthyle ;
c) un groupement C₃-C₄-alkyle ramifié saturé, préférablement isopropyle et isobutyle.

4. Procédé selon l'une des revendications précédentes, dans lequel les composés de formule (I) sont choisis parmi :
| Structure | Composé n° | Nom chimique |
|---|---|---|
| 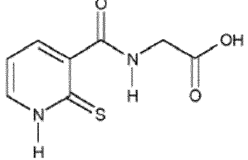 | 1 | N-[(2-thioxo-1,2-dihydropyridin-3-yl)carbonyl]glycine |
| 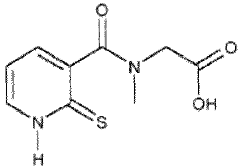 | 2 | N-méthyl-N-[(2-thioxo-1,2-dihydropyridin-3-yl)carbonyl]glycine |
| 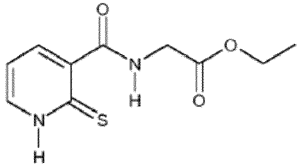 | 3 | N-[(2-thioxo-1,2-dihydropyridin-3-yl)carbonyl]glycinate d'éthyle |
| 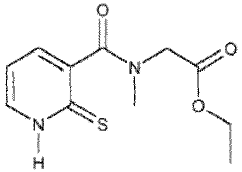 | 4 | N-méthyl-N-[(2-thioxo-1,2-dihydropyridin-3-yl)carbonyl]glycinate d'éthyle |
ainsi que les tautomères de ceux-ci, les sels de ceux-ci, les solvates de ceux-ci et les isomères optiques de ceux-ci, et les racémates de ceux-ci, seuls ou sous forme de mélange.

5. Procédé selon la revendication 1, dans lequel le composé de formule (I) est choisi parmi les composés suivants :
| Structure | Composé n° | Nom chimique |
|---|---|---|
| 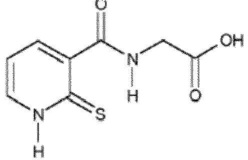 | 1 | N-[(2-thioxo-1,2-dihydropyridin-3-yl)carbonyl]glycine |
| 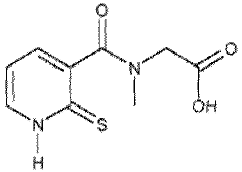 | 2 | N-méthyl-N-[(2-thioxo-1,2-dihydropyridin-3-yl)carbonyl]glycine |

6. Procédé selon l'une des revendications précédentes, dans lequel le composé de formule (I) est présent, seul ou sous forme de mélange, dans la composition selon une quantité comprise entre 0,01% et 10% en poids, préférablement entre 0,1% et 5% en poids, et en particulier entre 0,5% et 3% en poids, par rapport au poids total de la composition.

7. Procédé selon l'une des revendications précédentes, dans lequel la composition comprend au moins un adjuvant choisi dans le groupe constitué par : l'eau ; les solvants organiques, en particulier les C₁-C₆-alcools, et les esters d'acides C₂-C₁₀-carboxyliques ; les huiles à base de carbone et/ou de silicone d'origine minérale, animale et/ou végétale ; les cires, les pigments, les charges, les colorants, les agents tensioactifs, les émulsifiants, les co-émulsifiants ; les agents actifs cosmétiques ou dermatologiques, les agents anti-UV, les polymères, les agents gélifiants hydrophiles ou lipophiles, les épaississants, les conservateurs, les parfums, les bactéricides, les céramides, les absorbeurs d'odeurs, les antioxydants.

8. Procédé selon l'une des revendications précédentes, dans lequel la composition comprend au moins un agent actif choisi dans le groupe constitué par : les agents desquamants ; les agents adoucissants, les agents photo-protecteurs organiques ou inorganiques, les hydratants ; les agents de dépigmentation ou de pro-pigmentation ; les agents anti-glycation ; les inhibiteurs de NO synthase ; les agents destinés à la stimulation de la synthèse de macromolécules dermiques ou épidermiques et/ou à la prévention de leur dégradation ; les agents destinés à la stimulation de la prolifération des fibroblastes et/ou des kératinocytes ou à la stimulation de la différentiation des kératinocytes ; les myorelaxants et/ou les agents de décontraction dermique ; les agents tenseurs ; les agents anti-pollution et/ou les agents antiradicalaires ; les agents agissant sur la circulation capillaire ; les agents agissant sur le métabolisme énergétique de cellules ; et des mélanges de ceux-ci.

9. Utilisation cosmétique non thérapeutique d'un composé de formule (I) tel que défini selon l'une quelconque des revendications 1 à 6, comme agent destiné à la dépigmentation, l'éclaircissement et/ou la décoloration de matières kératiniques.

10. Composés de formule (II) : 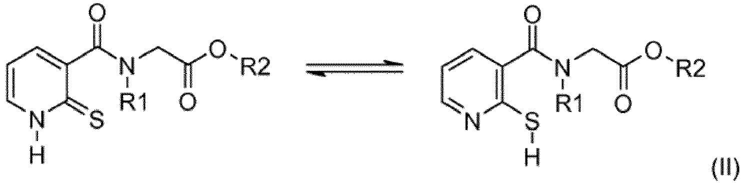 dans lequel
R1 désigne un radical choisi parmi :
a) un atome d'hydrogène ;
b) un groupement C₁-C₆-alkyle linéaire saturé ;
R2 désigne un radical choisi parmi :
a) un atome d'hydrogène ;
b) un groupement C₁-C₁₀-alkyle linéaire saturé ;
c) un groupement C₃-C₁₀-alkyle ramifié saturé ;
d) un groupement C₁-C₆-phénylalkyle tel que benzyle ;
ainsi que les tautomères de ceux-ci, les sels de ceux-ci, les solvates de ceux-ci et les isomères optiques de ceux-ci, et les racémates de ceux-ci, seuls ou sous forme de mélange,
à l'exception des deux composés (a) et (b) suivants et des tautomères de ceux-ci :

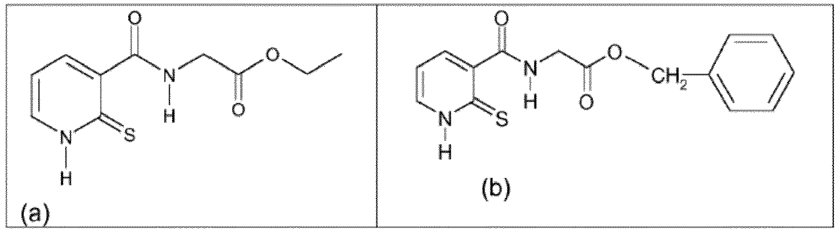

11. Composés de formule (II) selon la revendication précédente, pour lesquels :
R1 désigne un radical choisi parmi :
a) un atome d'hydrogène ;
b) un radical C₁-C₄-alkyle linéaire saturé, et préférablement méthyle ;
R2 désigne un radical choisi parmi :
a) un atome d'hydrogène ;
b) un groupement C₁-C₆-alkyle linéaire saturé ;
c) un groupement C₃-C₆-alkyle ramifié saturé,
ainsi que les tautomères de ceux-ci, les sels de ceux-ci, les solvates de ceux-ci et les isomères optiques de ceux-ci, et les racémates de ceux-ci, seuls ou sous forme de mélange,
à l'exception du composé (a) suivant et du tautomère de celui-ci :

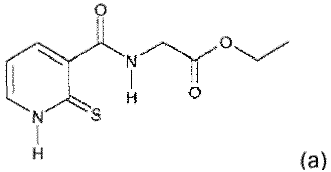

12. Composés de formule (II) selon la revendication 10 ou bien 11, choisis parmi :
| Structure | Composé n° | Nom chimique |
|---|---|---|
| 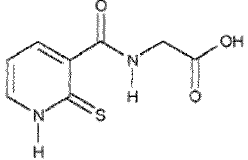 | 1 | N-[(2-thioxo-1,2-dihydropyridin-3-yl)carbonyl]glycine |
| 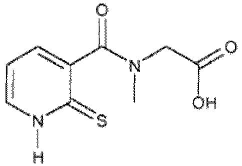 | 2 | N-méthyl-N-[(2-thioxo-1,2-dihydropyridin-3-yl)carbonyl]glycine |
| 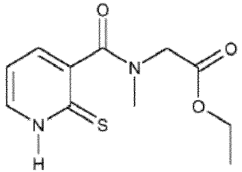 | 4 | N-méthyl-N-[(2-thioxo-1,2-dihydropyridin-3-yl)carbonyl]glycinate d'éthyle |
ainsi que les tautomères de ceux-ci, les sels de ceux-ci, les solvates de ceux-ci et les isomères optiques de ceux-ci, et les racémates de ceux-ci, seuls ou sous forme de mélange.

13. Composition, en particulier une composition cosmétique, comprenant au moins un composé de formule (II) selon l'une des revendications 10 à 12.

14. Composition selon la revendication précédente, **caractérisée en ce que** ledit composé de formule (II) est présent selon une quantité comprise entre 0,01% et 10% en poids, préférablement entre 0,1% et 5% en poids, et en particulier entre 0,5% et 3% en poids, par rapport au poids total de la composition.

15. Procédé de préparation de composés de formules (I) et (II) tels que définis selon l'une quelconque des revendications 1 à 12, selon le schéma suivant : 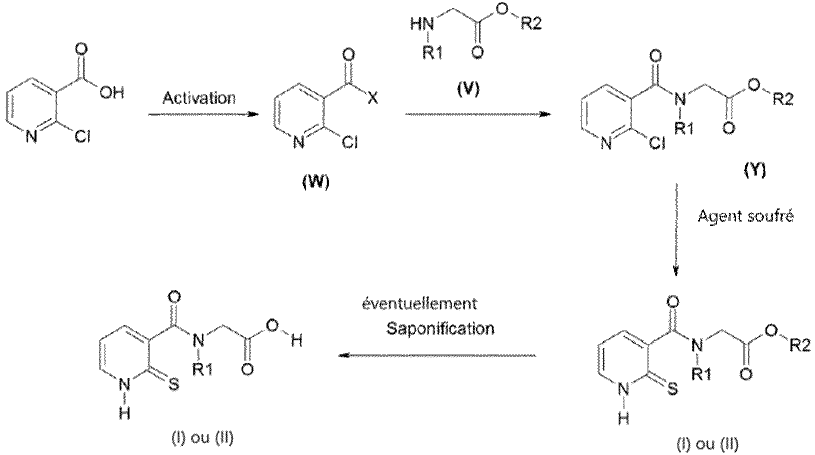 où R1, R2 et X revêtent les mêmes significations que celles précédemment définies.

16. Procédé de préparation selon la revendication précédente, qui comprend les étapes suivantes :
(i) l'étape de préparation d'un composé de formule (W) 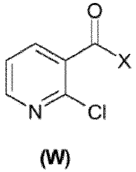 où X forme un halogénure d'acide, un anhydride mixte, un carbamimidate ou un acylphosphonate, par activation du groupement carboxylique de l'acide 2-chloronicotinique en présence d'un agent d'activation des acides carboxyliques selon les méthodes classiques pour l'activation des acides ; puis
(ii) l'étape de réaction du composé (W) avec une amine de formule (V) 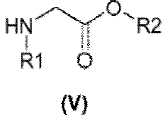 où R1 et R2 revêtent les mêmes significations que celles décrites selon la revendication 1, de façon à former le composé de formule (Y) : 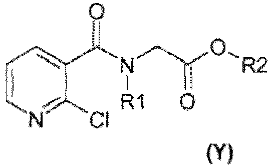 puis
(iii) l'étape d'échange entre le chlore et le soufre à l'aide de réactifs tels que le disulfite de sodium, la thiourée, le thiosulfate de sodium ou l'acide thioacétique, de façon à former un composé de formules (I) et (II), puis éventuellement
(iv) pour les composés de formules (I) et (II) où le radical R2 représente hydrogène, lesdits composés peuvent être obtenus soit directement, soit au moyen d'une étape supplémentaire de saponification des esters correspondants à l'aide de bases inorganiques suite à l'acidification.
